# EUROPÄISCHE PATENTSCHRIFT

(11) **EP 3 200 720 B1**
(45) Veröffentlichungstag und Bekanntmachung des Hinweises auf die Patenterteilung: **09.12.2020**
(21) Anmeldenummer: 15778225.1
(22) Anmeldetag: 24.09.2015
(51) Int. Cl.: A61C 13/00, A61C 13/01

(54) **FORM ZUR HERSTELLUNG EINES VORGEFORMTEN PROTHESENBASISROHLINGS**
MOLD FOR PRODUCING A PREFORMED BLANK FOR A PROSTHESIS BASE
MOULE POUR LA PRÉPARATION D'UNE ÉBAUCHE PREFORMÉE D'UNE BASE DE PROTHÈSE

(30) Priorität: 01.10.2014 DE 102014114278
(43) Veröffentlichungstag der Anmeldung: 09.08.2017
(73) Patentinhaber: Kulzer GmbH, 63450 Hanau (DE)
(72) Erfinder: RUPPERT, Klaus, 63477 Maintal (DE); ECK, Michael, 61389 Schmitten (DE)
(74) Vertreter: Schultheiss & Sterzel Patentanwälte PartG mbB
(86) Internationale Anmeldenummer: PCT/EP2015/071980
(87) Internationale Veröffentlichungsnummer: WO 2016/050600

(56) Entgegenhaltungen:
- WO-A1-2014/134640
- WO-A1-2014/191286
- GB-A- 426 744
- US-A1- 2008 153 069

## Beschreibung

Die Erfindung betrifft eine Form zur Herstellung eines vorgeformten Prothesenbasisrohlings, wobei der vorgeformte Prothesenbasisrohling zur Herstellung einer Prothesenbasis für eine Dentalprothese mit einem subtraktiven CAM-Verfahren vorgesehen ist.

Die Erfindung betrifft auch ein Verfahren zur Herstellung eines Prothesenbasisrohlings mit einer solchen Form und einen Prothesenbasisrohling hergestellt mit einem solchen Verfahren oder mit einer solchen Form.

Die Erfindung betrifft somit im Endeffekt die Herstellung von partiellen Kunststoffprothesen (Teilprothesen) und totalen Kunststoffprothesen (Totalprothesen) für den zahnmedizinischen Bereich beziehungsweise von Prothesenbasen hierfür, die maschinell im CAM-Verfahren (CAM - Computer-Aided Manufacturing, Deutsch: rechner-unterstützte Fertigung) produziert werden. Bevorzugt werden die Dentalprothesen und die Prothesenbasen hierzu computergestützt mit CAD-Verfahren (CAD - Computer-Aided Design, Deutsch: rechnerunterstützte Konstruktion) konstruiert. Dabei wird zunächst eine Prothesenbasis hergestellt, die später auf dem zahnlosen oder bereichsweise zahnlosen Zahnfleisch des Kieferbogens eines Patienten aufliegt. Die Prothesenzähne werden anschließend individuell gefertigt und in die Prothesenbasis eingesetzt und dort befestigt. Die Prothesenbasis mit den aufgestellten Prothesenzähnen bildet dann die fertige Dentalprothese.

Der gängige Weg ist die analoge Erstellung von Dentalprothesen. Zur Herstellung der Prothesenbasis wird derzeit also meist ein analoges Verfahren verwendet, bei dem zunächst ein Abdruck des zahnlosen Kiefers des Patienten genommen wird. Aus diesem Abdruck wird eine Form gefertigt, die mit einem zahnfleischfarbenen Kunststoff ausgegossen wird. Nach Aushärtung des Kunststoffs wird dieser nachbearbeitet, um die gewünschte Form zu erhalten. Anschließend werden die separat hergestellten Prothesenzähne eingesetzt, die ebenfalls derzeit meist analog hergestellt werden.

Zur Herstellung der Prothese werden Prothesenzähne manuell und einzeln auf einer Wachsbasis aufgestellt. Diese Wachsprothese wird im nächsten Schritt in einer Küvette mit Gips eingebettet, um dann nach Aushärten des Gipses die Wachsbasis mit heißem Wasser herauszuwaschen und einen Hohlraum für den Prothesenkunststoff zu schaffen. Die Prothesenzähne verbleiben dabei im Gips. Ein entsprechender Prothesenwerkstoff wird in den Hohlraum injiziert oder gestopft und man erhält nach Aushärtung des Kunststoffs die Prothese beziehungsweise den fertigen Prothesenzahn.

Bei der Aufstellung der konfektionierten Zähne werden diese der jeweiligen Mundsituation des Patienten vom Zahntechniker und gegebenenfalls auch vom Zahnarzt angepasst und beschliffen.

Ein solches Verfahren ist aus der WO 91/07141 A1 bekannt, bei dem eine Prothesenbasis auf der Basis eines Abdrucks aus einem Kunststoffblock gefräst wird. Es gibt bereits erste Verfahren, wie beispielsweise die aus der DE 10 2009 056 752 A1 oder der WO 2013 124 452 A1 bekannten Verfahren, bei denen die Teil- beziehungsweise Totalprothese digital aufgestellt und über CAD-CAM-Verfahren produziert wird. Die FR 2 582 932 A1 schlägt vor, dass eine Prothesenbasis mit Hilfe eines Wachsabdrucks und einer 3D-Kopierfräse erzeugt wird, dieses Verfahren ist aufwendiger als die modernen CAD-CAM-Verfahren. Aus der WO 2013 068 124 A2 ist ein Fräsblock mit vorgeformten Prothesenzähnen bekannt. Nachteilig ist hieran, dass die Prothesenzähne aus dem gleichen Material wie die Basis bestehen und bei einer Beschädigung das ganze Gebiss getauscht werden muss und die fertig konstruierte Prothese nur mit großem Aufwand an die Bedürfnisse des Patienten (beispielsweise bezüglich der Zahnfarbe oder der Okklusion und Zahnstellung) angepasst werden kann. Ein weiterer Nachteil liegt darin, dass die Basis und der Prothesenzahn (beziehungsweise die Prothesenzahnkrone) unterschiedliche Anforderungen haben aber aus dem gleichen Material gefertigt sind. So soll der Zahn vorrangig kauen und dabei unbeschädigt bleiben. Die Basis sollte hingegen die auftretenden Kräfte über die Schleimhaut verteilen. Dies kann bei der Verwendung nur eines Materials zu Problemen führen. Die WO 2014/134 640 A1 offenbart ein Verfahren zur Herstellung einer Dentalprothese, bei dem zur Herstellung der Prothesenbasis ein Hohlraum mit einem Kunststoff ausgegossen wird. Aus der GB 426 744 A ist ein Prothesenbasisrohling bekannt, der mit einer Presse umgeformt wird. Dabei fließt das Material aufgrund von Druck in die freien Teile einer Form der Prothesenbasis.

Aus der DE 20 2006 006 286 U1 ist ein runder Rohling zur Herstellung einer Zahnprothese bekannt. Solche Rohlinge sind im Prinzip massive Polymethylmethacrylat-Scheiben (PMMA-Scheiben). Dieser Rohling (der sogenannten Ronde) wird in eine CAM-Fräsmaschine eingespannt und auf Basis eines CAD-Modells automatisch abgefräst. Aktuelle Verfahren fräsen aus einem solchen vollen Block (der Ronde) eine lediglich 2 mm bis 3 mm dicke Prothesenbasis. Nachteile sind einerseits eine sehr hohe Materialverschwendung, die je nach anatomischer Situation des jeweiligen Patienten problemlos über 90% betragen kann. Das führt andererseits zu einer zeitintensiven Bearbeitungszeit durch die CAM-Vorrichtung, da sehr viel Material abgetragen werden muss. Darüber hinaus ist die Abnutzung der jeweiligen Fräswerkzeuge relativ hoch.

Nachteilig ist hieran ferner, dass es relativ lange dauert, bis die Prothesenbasis hergestellt ist. Zudem nutzt sich durch die Bearbeitung des Rohlings der Fräskopf ab und muss in regelmäßigen Abständen erneuert werden. Das abgefräste Material (die Frässpäne) muss entsorgt oder recycelt werden.

Die Aufgabe der Erfindung besteht also darin, die Nachteile des Stands der Technik zu überwinden. Insbesondere soll eine Möglichkeit gefunden werden, beziehungsweise ein Prothesenbasisrohling und ein Verfahren bereitgestellt werden, mit der beziehungsweise mit dem eine möglichst einfache, vollständige und kostengünstige Bearbeitung des Prothesenbasisrohlings beziehungsweise eine möglichst einfache, vollständige und kostengünstige Herstellung der Prothesenbasis möglich ist. Der Fräskopf beziehungsweise das Werkzeug zum Durchführen des subtraktiven CAM-Verfahrens soll dabei möglichst wenig beansprucht werden. Zudem soll die Herstellung der Prothesenbasis möglichst schnell erfolgen können. Gleichzeitig soll die herzustellende Prothesenbasis an die anatomischen Verhältnisse für verschiedene Patienten anpassbar sein.

Die Aufgaben der Erfindung werden gelöst durch eine Form zur Herstellung eines vorgeformten Prothesenbasisrohlings, wobei der vorgeformte Prothesenbasisrohling zur Herstellung einer Prothesenbasis für eine Dentalprothese mit einem subtraktiven CAM-Verfahren vorgesehen ist, wobei die Form eine innere Oberfläche aufweist, die ein Negativ des zu erzeugenden Prothesenbasisrohlings ist, wobei die innere Oberfläche ein Negativ eines Zahnbogens oder eines Zahnbogenabschnitts darstellt, wobei der Zahnbogen oder der Zahnbogenabschnitt eine Verdickung in okklusaler Richtung aufweist, und die Form aus zumindest zwei Teilen besteht, die voneinander lösbar aneinander befestigt sind oder die lösbar aneinander befestigbar sind, wobei die innere Oberfläche der Form neben dem Zahnbogen oder dem Zahnbogenabschnitt zusätzlich Stege und eine Halterung für den Prothesenbasisrohling definiert, wobei die Stege die Halterung mit dem Zahnbogen oder dem Zahnbogenabschnitt verbinden.

Abweichend von dem üblichen Verständnis wird vorliegend unter einen Zahnbogen nicht der Bogen der Prothesenzähne verstanden, sondern der auf dem Kiefersattel ausgebildete Teil der Prothese, die das Zahnfleisch nachbildet, in dem die Prothesenzähne befestigt werden sollen.

Die Verdickung in okklusaler Richtung ist notwendig, um die Papillen zwischen den Auflageflächen für die Prothesenzähne in dem Prothesenbasisrohling ausformen zu können. Da die Prothesenzähne je nach Patient individuell an verschiedenen Orten eingesetzt werden müssen, bietet der mit der erfindungsgemäßen Form geformte Prothesenbasisrohling ausreichend Raum beziehungsweise Material, um die Papillen (die Prothesenbereiche für das Zahnfleisch zwischen den Zähnen) an einer beliebigen Stelle ausformen zu können. Die Verdickung liegt erfindungsgemäß in Okklusionsrichtung auf dem Zahnbogen oder auf dem Zahnbogenabschnitt auf.

Dass ein Negativ eines Zahnbogens oder Zahnbogenabschnitts in der Form enthalten ist, bedeutet, dass die Form eine Aufnahme bildet, so dass das Material für den zu erzeugenden Zahnbogen des Prothesenbasisrohlings oder den zu erzeugenden Zahnbogenabschnitt des Prothesenbasisrohlings für die Teil-Prothesenbasis bereits in einer Materialverdickung bei Verwenden der Form, beziehungsweise in dem mit der Form erzeugten Prothesenbasisrohling enthalten ist. Dazu sind die Abmessungen des Zahnbogens oder des Zahnbogenabschnitts größer als eine Gruppe typischer zu fertigender Zahnbögen von Prothesenbasen einschließlich üblicher Toleranzen. Der Zahnbogen umfasst den Zahnbogen der Prothesenbasis aber nicht der einzusetzenden Prothesenzähne. Nach Einsetzen der Prothesenzähne kann das Gebiss, das mit dem aus einer solchen Form erzeugten Prothesenbasisrohling hergestellt wurde, also höher sein, als die Verdickung des Zahnbogens des unbearbeiteten Prothesenbasisrohlings.

Bei einer erfindungsgemäßen Form kann auch vorgesehen sein, dass sich die innere Oberfläche der Form durch einen Austausch von Teilen anpassen lässt, wobei die gegeneinander austauschbaren Teile einen abweichenden Teilbereich der inneren Oberfläche der Form zur Herstellung unterschiedlicher Prothesenbasisrohlinge aufweisen.

Hierdurch ist die Form als Set von Teilen zur Herstellung unterschiedlicher Prothesenbasisrohlinge geeignet, die dadurch bereits an bestimmte Formtypen für Prothesenbasen angepasst sind.

Ferner kann vorgesehen sein, dass die Form mehrere austauschbare Teile und/oder innerhalb der Form einsetzbare Einlagen aufweist, die die innere Oberfläche bei einer Verwendung der Teile und/oder Einlagen zum Zusammensetzen der Form verändern.

Hierdurch wird eine hohe Variabilität der Form erreicht und eine noch genauere Anpassung des zu erzeugenden Prothesenbasisrohlings an die Situation beim Patienten möglich. Die Form bildet dann genaugenommen ein Set von Teilen unterschiedlich zusammensetzbarer Formen. Gegebenenfalls können Prothesenbasisrohlinge in verschiedenen Größenabstufungen hergestellt werden.

Zur einfacheren Bearbeitung mit vorhandenen CAM-Vorrichtungen ist vorgesehen, dass die innere Oberfläche der Form neben dem Zahnbogen oder dem Zahnbogenabschnitt zusätzlich Stege und eine Halterung für den Prothesenbasisrohling definiert, wobei die Stege die Halterung mit dem Zahnbogen verbinden.

Mit einer solchen Halterung kann der Prothesenbasisrohling in CAM-gesteuerte Fräsen oder andere Vorrichtungen zum subtraktiven Herstellen von Prothesenbasen eingespannt werden. Bevorzugt ist die innere Oberfläche der Form derart geformt, dass bei dem mit der Form hergestellten Prothesenbasisrohling die Halterung ausschließlich über die Stege mit dem Zahnbogen oder dem Zahnbogenabschnitt verbunden ist.

Bei einer Ausführung der Form, die zur Herstellung einer Halterung als Teil des Prothesenbasisrohlings geeignet ist, kann vorgesehen sein, dass die Halterung, die durch die innere Oberfläche der Form definiert ist, einen Ring um den zu erzeugenden Zahnbogen oder Zahnbogenabschnitt bildet, wobei bevorzugt der Zahnbogen oder der Zahnbogenabschnitt über wenigstens drei Stege mit dem Ring verbunden ist.

Hierdurch kann der Prothesenbasisrohling mit den üblichen Halterungen für Ronden verwendet werden. Die Verwendung von zumindest drei Stegen hat den Vorteil, dass eine ausreichende Stabilität des Zahnbogens oder des Zahnbogenabschnitts im Ring erzeugt wird, so dass sich der Zahnbogen oder der Zahnbogenabschnitt bei der mechanischen Belastung beim Bearbeiten des Prothesenbasisrohlings nicht gegen den Ring drehen kann. Theoretisch ist der gleiche Effekt auch durch zwei oder sogar nur einen ausreichend breiten Steg zu erreichen. Zumindest drei kompaktere Stege werden aber erfindungsgemäß bevorzugt, da sich die Stege dann leichter trennen lassen und auch die Nachbearbeitung beim Entfernen der Stegansätze von der abgetrennten Prothesenbasis einfacher ist. Die Stege sollen im Durchmesser so klein wie möglich gehalten werden, um den Aufwand bei der Nacharbeitung so gering wie möglich zu halten.

Ferner kann vorgesehen sein, dass die zumindest zwei Teile der Form in zumindest einer Fugenebene aneinander in Passform anliegen, wobei alle der durch die Form zu erzeugenden Stege in der zumindest einen Fugenebene der Form angeordnet sind.

Hierdurch wird verhindert, dass sich der Prothesenbasisrohling durch die innere Oberfläche, insbesondere an den Stellen, die die Stege ausbilden sollen, unlösbar mit der Form verbindet. Wenn die Form aus drei Teilen zusammengesetzt wird, dann werden diese in zwei Fugenebenen aneinander gelegt. Wenn die Form aus vier Teilen zusammengesetzt wird, dann werden diese in zwei oder drei Fugenebenen aneinander gelegt. Bei weiteren Teilen sind entsprechend mehr Fugenebenen möglich. Die Fugenebenen müssen nicht geometrisch in einer flachen Ebene angeordnet sein. Stattdessen kann die Fugenebene auch eine gewellte, verdrehte, abgeknickte, gewölbte und/oder gebogene Ebene sein.

Mit einer Weiterbildung der erfindungsgemäßen Form wird vorgeschlagen, dass die innere Oberfläche der Form eine Ausnehmung oder einen Vorsprung definiert, der als Referenzmarkierung zur Bestimmung der Orientierung des Prothesenbasisrohlings verwendbar ist, wobei bevorzugt die innere Oberfläche der Form eine Ausnehmung oder einen Vorsprung auf der Halterung definiert, insbesondere auf dem Ring definiert.

Mit dieser Maßnahme kann die Orientierung des Prothesenbasisrohlings vollautomatisch bestimmt und definiert werden. Dies ist hilfreich, da der Prothesenbasisrohling bereits vorgeformt ist und also nur in einer bestimmten Lage und Orientierung oder in einem engen Bereich um diese Lage und Orientierung sinnvoll bearbeitet werden kann. Erfindungsgemäß bevorzugte Formen können sich dadurch auszeichnen, dass die Form aus Metall, Silikon oder Dubliergel besteht, bevorzugt aus Metall besteht, besonders bevorzugt aus Stahl, Aluminium oder Messing besteht.

Diese Materialien sind zur Herstellung der Prothesenbasisrohlinge gut geeignet. Metallische Formen sind bevorzugt, da sie sich besonders gut wiederverwenden lassen.

Es wird mit der Erfindung auch vorgeschlagen, dass die innere Oberfläche der Form beschichtet ist.

Hierdurch kann eine bessere Lösbarkeit des ausgehärteten beziehungsweise fertigen Prothesenbasisrohlings von der Form beziehungsweise deren Teilen erreicht werden. Die Beschichtung kann fest mit der inneren Oberfläche der Form verbunden sein oder vor jeder Herstellung eines Prothesenbasisrohlings neu aufgetragen werden. Alternativ zur Beschichtung kann die Oberfläche eine sehr geringe Oberflächenrauhigkeit aufweisen, die beispielsweise durch Elektropolitur erzeugt werden kann.

Gemäß einer Weiterbildung kann auch vorgesehen sein, dass die zumindest zwei Teile entlang einer Transversalebene aneinander gefügt sind oder aneinander zu fügen sind.

Hierdurch kann eine einfache Entformung des Prothesenbasisrohlings erreicht werden. Dies gilt insbesondere dann, wenn Stege zur Verbindung des Zahnbogens oder Zahnbogenabschnitts vorgesehen sind und diese ebenfalls in der Transversalebene angeordnet sind. Die Transversalebene ist dabei eine Ebene, in der der Kieferbogen der herzustellenden Prothesenbasis liegt.

Zur Vereinfachung der Herstellung der Prothesenbasisrohlinge kann des Weiteren vorgesehen sein, dass die innere Oberfläche der Teile der Form keinen geometrischen Körper bildet, der beim Entformen des Prothesenbasisrohlings das Lösen der Form von dem ausgehärteten Prothesenbasisrohling verhindert, wobei bevorzugt keine Unterschneidungen oder unter sich gehende Bereiche in der inneren Oberfläche der Teile der Form vorgesehen sind.

Hierdurch kann sichergestellt werden, dass sich die Teile der Form zerstörungsfrei und weitgehend ohne mechanische Belastung des hergestellten Prothesenbasisrohlings von dem ausgehärteten Prothesenbasisrohling lösen lassen.

Die der vorliegenden Erfindung zugrundeliegenden Aufgaben werden auch gelöst durch ein Verfahren zur Herstellung eines Prothesenbasisrohlings mit einer erfindungsgemäßen Form, bei dem ein Heißpolymerisat oder ein Kaltpolymerisat in die zumindest zweiteilige, zusammengesetzte Form gefüllt wird, anschließend das Heißpolymerisat oder das Kaltpolymerisat aushärtet und daran anschließend die Teile der Form von dem ausgehärteten Prothesenbasisrohling gelöst werden.

Dabei kann vorgesehen sein, dass der Prothesenbasisrohling zumindest bereichsweise entgratet wird, nachdem die Teile der Form abgelöst wurden.

Bevorzugt erfolgt die Aushärtung unter erhöhtem Druck zur Vermeidung von Blasen im Material.

Des Weiteren kann vorgesehen sein, dass ein Heißpolymerisat in die zumindest zweiteilige, zusammengesetzte Form aus Metall mit einer Stopf-Press-Technik oder einer Injektionstechnik gefüllt wird.

Alternativ kann vorgesehen sein, dass ein Kaltpolymerisat in die zumindest zweiteilige, zusammengesetzte Form aus Metall, Silikon oder Dubliergel mit einer Gieß-Technik oder einer Injektionstechnik gefüllt wird.

Ein Verfahren kann zur Herstellung einer Prothesenbasis zur Herstellung einer Totaldentalprothese oder wenigstens einer Teildentalprothese mit einem Prothesenbasisrohling die folgenden Verfahrensschritte aufweisen:
1) Befestigen des Prothesenbasisrohlings in einer CAM-Vorrichtung zum Abtragen von Material des Prothesenbasisrohlings mit einem CAM-Verfahren und
2) Abtragen von Material des Prothesenbasisrohlings mit der CAM-Vorrichtung auf der Basis eines berechneten CAD-Modells.

Aus dem erfindungsgemäß hergestellten Prothesenbasisrohling kann entweder eine Totalprothese für einen Kieferteil gefräst beziehungsweise geformt werden oder es können mehrere Teilprothesen aus einem Prothesenbasisrohling gefräst beziehungsweise geformt werden.

Mit dem erfindungsgemäßen Verfahren wird auch vorgeschlagen, dass die Form, die Teile der Form und/oder Einlagen der Form zur Herstellung des Prothesenbasisrohlings aus einer Vielzahl von Formen, Teilen und/oder Einlagen ausgewählt wird, wobei die Auswahl rechnerisch auf der Grundlage des CAD-Modells der zu erzeugenden Prothesenbasis und den bekannten Abmessungen aller Formen, Teile der Formen und Einlagen der Formen erfolgt, wobei bevorzugt über eine Ausgabeeinrichtung eines Computers alle möglichen Formen und/oder Zusammenstellungen der Teile der Formen und/oder Zusammenstellungen der Einlagen der Formen als Auswahl vorgeschlagen werden und/oder über eine Eingabeeinrichtung ausgewählt werden können.

Ferner kann erfindungsgemäß auch vorgesehen sein, dass die Form ausgewählt wird oder die Kombination von Teilen und/oder Einlagen einer Form ausgewählt werden aus der Menge der Formen, Teile und/oder Einlagen, deren innere Oberfläche die äußeren Abmessungen der zu erzeugenden Prothesenbasis, die durch das CAD-Modell berechnet ist, vollständig aufnehmen können, wobei bevorzugt die vorgeschlagene Reihenfolge, insbesondere die angezeigte oder dargestellte Reihenfolge, der ausgewählten Formen, Teile und/oder Einlagen bestimmt wird durch die Volumendifferenz zwischen der inneren Oberfläche der zusammengesetzten Formen, Teile und/oder Einlagen und dem CAD-Modell der zu erzeugenden Prothesenbasis.

Es kann erfindungsgemäß auch vorgesehen sein, dass automatisch die Form oder die Kombination von Teilen und/oder Einlagen einer Form vorgeschlagen wird oder verwendet wird, die die geringste Volumendifferenz zu dem CAD-Modell der zu erzeugenden Prothesenbasis aufweist.

Die der Erfindung zugrundeliegenden Aufgaben werden ferner gelöst durch einen Prothesenbasisrohling hergestellt mit einem erfindungsgemäßen Verfahren oder hergestellt mit Hilfe einer erfindungsgemäßen Form.

Dabei kann vorgesehen sein, dass der Prothesenbasisrohling Stege aufweist, die mit einem Ring als Halterung verbunden sind und die den Zahnbogen oder den Zahnbogenabschnitt im Inneren des Rings halten.

Bei erfindungsgemäßen Prothesenbasisrohlingen bleibt ein gewisser Spielraum, um unterschiedliche Prothesenbasen aus dem Prothesenbasisrohling fertigen zu können. Es reicht aus, wenn die Lage der zu erzeugenden Prothesenbasis in dem Prothesenbasisrohling auf 10 mm genau ist, bevorzugt auf 5 mm genau ist, und die Orientierung der zu erzeugenden Prothesenbasis in dem Prothesenbasisrohling auf 10° genau ist, bevorzugt auf 5° genau ist. Die Orientierung bezieht sich dabei auf mögliche Verkippungswinkel der zu erzeugenden Prothesenbasis relativ zum vorgeformten Prothesenbasisrohling.

Es kann erfindungsgemäß vorgesehen sein, dass der Prothesenbasisrohling genau eine Symmetrieebene oder zwei senkrecht zueinander orientierte Symmetrieebenen aufweist, wobei genau eine Symmetrieebene bevorzugt ist. Bevorzugt kann dabei vorgesehen sein, dass die eine Symmetrieebene oder eine der Symmetrieebenen der Sagittalebene der zu erzeugenden Prothesenbasis entspricht.

Es kann erfindungsgemäß auch vorgesehen sein, dass der Prothesenbasisrohling auf der Seite zur Auflage auf dem Zahnfleisch eine gewölbte Vertiefung als Vorformung einer Aufnahme eines zahnlosen Kiefersattels oder eines Teils eines zahnlosen Kiefersattels aufweist, wobei die Vertiefung sich entlang des Zahnbogens oder Zahnbogenabschnitts erstreckt.

Ferner kann vorgesehen sein, dass der Prothesenbasisrohling aus Polymethylmethacrylat (PMMA), Polyetherketon (PEK), Polyetheretherketon (PEEK), Polyamid (PA), Polycarbonat (PC) oder Polyurethan (PU) oder einer Kombination hieraus besteht beziehungsweise mit diesen Materialien oder einer Kombination hiervon erzeugt wird. Diese Materialien sind für die Bearbeitung des Prothesenbasisrohlings mit CAM-Verfahren besonders gut geeignet. Zudem lassen sich daraus auch ästhetisch passende Prothesenbasen fertigen.

Der Erfindung liegt die überraschende Erkenntnis zugrunde, dass es mit der Form in einfacher Weise gelingt, einen kompakten Prothesenbasisrohling zu erzeugen, dessen äußere Abmessungen und Geometrie bereits an die äußeren Abmessungen und die Geometrie der aus dem Prothesenbasisrohling zu erzeugenden Prothesenbasis angepasst ist, so dass aus den mit der Form oder dem Verfahren hergestellten Prothesenbasisrohlingen schnell und durch ein Abtragen von nur geringen Mengen des Materials die gewünschten Prothesenbasen zu fertigen sind.

Mit den vorgeformten Prothesenbasisrohlingen gelingt es so, ein schnelleres und effizienteres Verfahren zur Herstellung von Prothesenbasen zu ermöglichen, bei denen die Werkzeuge der CAM-Vorrichtung geschont werden und der Materialverlust durch die subtraktiven CAM-Verfahren minimiert werden kann. Durch die geeignete Form bleiben die Prothesenbasisrohlinge dabei ausreichend flexibel, um problemlos an die unterschiedlichen Mundsituationen verschiedener Patienten und an die anderen Anforderungen für die zu erzeugende Prothesenbasis angepasst zu werden.

Das Verfahren und die mit der Form erzeugten Prothesenbasisrohlinge sind zur Bearbeitung mit CAD/CAM-Verfahren vorgesehen und hierfür besonders geeignet.

Der dieser Erfindung zu Grunde liegende Lösungsansatz geht bevorzugt von diversen Formen beziehungsweise Teilen und Einlagen für Formen zum Herstellen verschiedener vorgeformter Prothesenbasisrohlinge aus, die die oben genannten Nachteile dadurch eliminieren, dass es sich nicht um Vollkörper beziehungsweise Vollscheiben sondern um vorgeformte Rohlinge handelt. Die Erfindung zeichnet sich dadurch aus, dass sie - ähnlich Abformlöffeln - den menschlichen Kiefer anhand anatomischer Merkmale in Kategorien beziehungsweise Größen einteilt und dementsprechend weitestgehend vorgeformte Prothesenbasisrohlinge bereitstellt. Bei der Verwendung unterschiedlicher austauschbarer Teile der Formen kann der zu erzeugende Prothesenbasisrohling bereits in gewissem Umfang an die Mundsituation oder an den Typ des Patienten angepasst werden und so die abzutragende Menge des Materials weiter reduziert werden. Die in einem CAD-CAM-System verwendete Software kann dabei für die jeweilige Patientensituation eine passende Form beziehungsweise passende Teile der Form auswählen und übernimmt das sogenannte Nesting in den vorgeformten Prothesenbasisrohlingen (das best-mögliche Positionieren der virtuellen Form der zu erzeugenden Prothesenbasis in dem Prothesenbasisrohling). Die Prothesenbasisrohlinge können bevorzugt aus unterschiedlichen Materialien wie Kunststoffen oder Wachs hergestellt sein.

Die Vorteile der erfindungsgemäßen Form und des erfindungsgemäßen Verfahren, beziehungsweise der mit solchen Verfahren und Formen erzeugten Prothesenbasisrohlinge liegen unter anderem in den geringeren Fräszeiten, in einem geringeren Materialeinsatz an Prothesenbasismaterial und in einer Schonung der Fräswerkzeuge, das heißt in einem geringeren Verschleiß der Fräswerkzeuge im Vergleich zur Anwendung üblicher Fräsrohlinge.

Im Folgenden werden Ausführungsbeispiele der Erfindung anhand von sechs schematisch dargestellten Figuren erläutert, ohne jedoch dabei die Erfindung zu beschränken. Dabei zeigt:
- Figur 1:: eine schematische Aufsicht auf einen erfindungsgemäßen Prothesenbasisrohling;
- Figur 2:: eine schematische seitliche Ansicht einer erfindungsgemäßen Form zur Herstellung eines Prothesenbasisrohlings;
- Figur 3:: eine schematische Aufsicht auf die formgebende Oberfläche eines Teils der erfindungsgemäßen Form nach Figur 2;
- Figur 4:: eine schematische seitliche Ansicht eines erfindungsgemäßen Prothesenbasisrohlings;
- Figur 5:: eine schematische Aufsicht auf einen erfindungsgemäßen Prothesenbasisrohling; und
- Figur 6:: eine schematische Aufsicht auf einen erfindungsgemäßen Prothesenbasisrohling für eine Teilprothese.

In den Figuren werden auch bei unterschiedlichen Ausführungen für gleichartige Bauteile teilweise die gleichen Bezugszeichen verwendet.

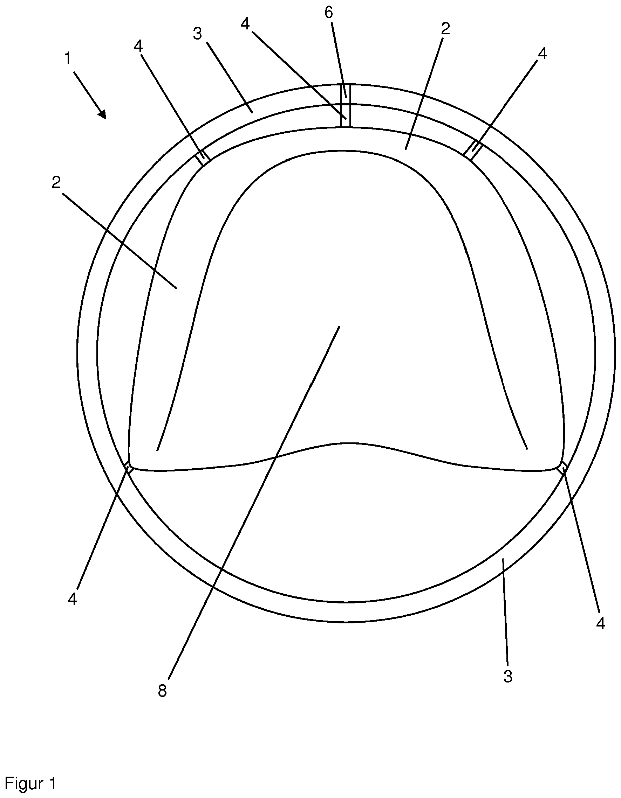

Figur 1 zeigt eine schematische Aufsicht auf einen erfindungsgemäßen Prothesenbasisrohling 1, der mit einem erfindungsgemäßen Verfahren beziehungsweise einer erfindungsgemäßen Form hergestellt wurde. Der Prothesenbasisrohling 1 besteht aus einem Kunststoff, bevorzugt aus einem Polymer. Randseitig weist der innere Bereich des Prothesenbasisrohlings 1 eine parabelförmige Verdickung auf, die einen vorgeformten Zahnbogen 2 mit Verdickung bildet. Der Prothesenbasisrohling 1 ist symmetrisch bezogen auf eine mittlere Ebene. Diese mittlere Ebene entspricht der Sagittalebene des Patienten, bei dem die mit dem Prothesenbasisrohling 1 hergestellte Dentalprothese eingesetzt wird.

Der Prothesenbasisrohling 1 weist einen Ring 3 auf, der den inneren Bereich des Prothesenbasisrohlings 1 umgibt. Der Ring 3 ist über fünf Stege 4 mit dem inneren Bereich des Prothesenbasisrohlings 1 verbunden. Der Ring 3 und die Stege 4 dienen ausschließlich der Halterung des Prothesenbasisrohlings 1 in einer standardisierten Halterung einer CAM-Vorrichtung, wie beispielsweise einer CAM-Vier-Achs-Fräse. Nach der Bearbeitung des inneren Bereichs des Prothesenbasisrohlings 1 zur Herstellung der Prothesenbasis, werden die Stege 4 getrennt und die dabei entstehenden Stümpfe der Stege 4 an der Prothesenbasis abgeschliffen. Die Prothesenbasis wird also nur aus dem inneren Bereich des Prothesenbasisrohlings 1 mit dem vorgeformten Zahnbogen 2 und mit einer Gaumenplatte 8 oder einer sublingualen Platte 8 gefertigt.

Der Scheitelpunkt des vorgeformten Zahnbogens 2 einer aus dem Prothesenbasisrohling 1 erzeugten Prothesenbasis wird später beim Patienten labial also lippenseitig angeordnet sein, wenn aus dem Prothesenbasisrohling 1 eine Totaldentalprothese gefertigt wird oder eine Teilprothese gefertigt wird, die zumindest die mittleren Schneidezähne umfasst.

Der gezeigte Prothesenbasisrohling 1 ist sowohl zur Herstellung einer Teilprothesenbasis oder mehrerer Teilprothesenbasen oder einer Totalprothesenbasis für einen Oberkiefer als auch für einen Unterkiefer geeignet. Es ist aber auch möglich, stärker vorgeformte Prothesenbasisrohlinge 1 herzustellen, bei denen zwischen Oberkiefer und Unterkiefer unterschieden wird. Zudem oder alternativ können eine Vielzahl verschiedener Formen zur Herstellung unterschiedlicher Prothesenbasisrohlinge 1 vorgehalten werden oder als Set vorgesehen sein, die sich durch die Krümmung der Zahnbögen 2 und deren Breite unterscheiden und aus denen die passende Form zur Herstellung eines Prothesenbasisrohlings 1 ausgewählt werden kann.

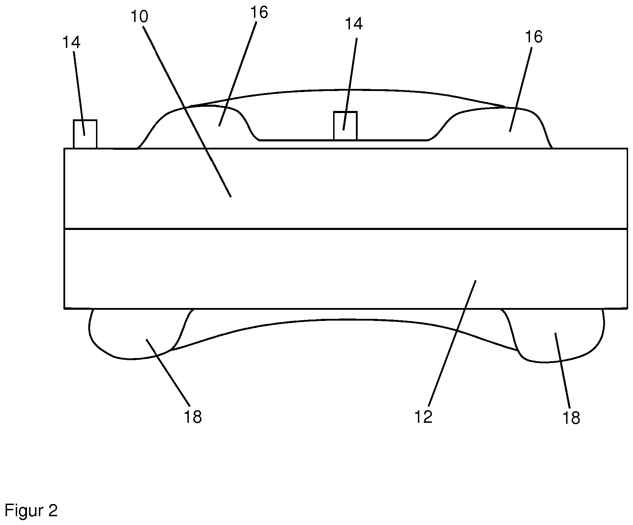

Figur 2 zeigt eine schematische seitliche Ansicht einer erfindungsgemäßen Form zur Herstellung eines Prothesenbasisrohlings umfassend zwei Teile 10, 12. Die beiden Teile 10, 12 werden derart aneinander gelegt beziehungsweise aneinander befestigt, dass sich im Inneren der Form eine innere Oberfläche ausbildet, die ein Negativ des Prothesenbasisrohlings 1 nach Figur 1 oder eines anderen Prothesenbasisrohlings bildet. Bezogen auf den Prothesenbasisrohling 1 nach Figur 1 bildet die innere Oberfläche der Form außenumfänglich den Ring 3 aus. In der Form sind zumindest zwei Einfüllstutzen 14 mit Öffnungen zum Einfüllen beziehungsweise Einpressen oder Injizieren von Material in die Form vorgesehen. Das gesamte Material zur Herstellung des Prothesenbasisrohlings muss auch nicht nur durch die Einfüllstutzen 14 in die Form eingetragen werden. Die Form kann auch oder zusätzlich vor dem zusammensetzen der beiden Teile 10, 12 mit dem Material gefüllt werden. Es kann zudem wenigstens eine Entlüftungsöffnung (nicht gezeigt) in den Formen enthalten sein, um es in der Form eingeschlossener Luft zu ermöglichen, beim Einpressen des Materials zu entweichen.

Die den Zahnbogen 2 und die Erhöhung beziehungsweise Verdickung darauf bildende innere Oberfläche ist von außen als Aufnahme 16 für die Erhöhung beziehungsweise Verdickung in okklusaler Richtung auf dem Zahnbogen am oberen Teil 10 der Form zu erkennen. Ebenso sind an der Unterseite der unteren Form 12 Aufnahmen 18 für die innere Oberfläche zur Ausformung der Auflage auf dem Zahnfleisch zu erkennen. Die Form könnte aber ebenso von außen zylindrisch aufgebaut sein.

Die beiden Teile 10, 12 der Form sind in einer Fugenebene (in Figur 2 die mittlere waagerechte Linie) miteinander verbunden. In der Ebene der Fugenebene liegen die beiden Teile 10, 12 mit Anlageflächen flächenbündig aneinander an. In der Ebene der Fugenebene sind auch alle auszuformenden Stege 4 des Prothesenbasisrohlings 1 angeordnet. Dadurch wird erreicht, dass sich die Teile 10, 12 der Form von dem Prothesenbasisrohling 1 trennen lassen, wenn der Prothesenbasisrohling 1 ausgehärtet ist, und nicht die Teile 10, 12 in die Stege 4 greifen und dadurch ein ablösen der Teile 10, 12 von dem ausgehärteten Prothesenbasisrohling 1 verhindern. In den Anlageflächen können Pins und Löcher (nicht gezeigt) beziehungsweise Nut und Feder (nicht gezeigt) vorgesehen sein, um eine eindeutige Orientierung der beiden Teile 10, 12 zueinander beim Befestigen der Teile 10, 12 aneinander zu erzwingen.

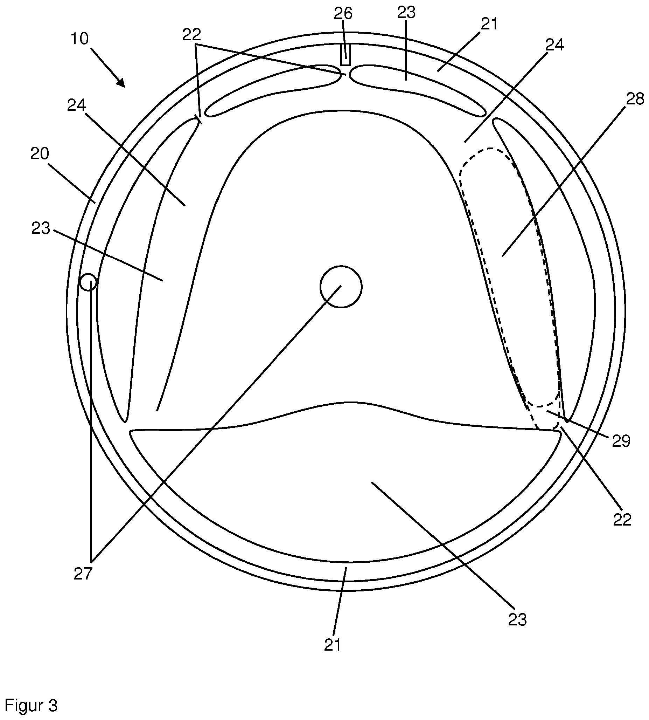

Figur 3 zeigt eine schematische Aufsicht in die formgebende Oberfläche des oberen Teils 10 der erfindungsgemäßen Form nach Figur 2. Der Teil 10 der Form hat einen Rand 20, der den gesamten Teil 10 der Form umschließt. Der Rand 20 bildet eine Fläche, die auf einem passenden Gegenstück, das heißt, auf einem passenden Rand des Teils 12 aufliegt und die Form nach außen abschließt. Direkt neben dem Rand 20 befindet sich eine Negativform für den Ring 21, also der Bereich der inneren Oberfläche der Form, der zum Ausformen des Rings 3 nach Figur 1 vorgesehen ist. Benachbart dazu befinden sich die Negativformen für die Stege 22, die zwischen fünf Auflageflächen 23 angeordnet sind. Die Negativformen für die Stege 22 sind dementsprechend der Bereich der inneren Oberfläche der Form, der zum Ausformen der Stege 4 nach Figur 1 vorgesehen ist. Die fünf Auflageflächen 23 liegen im zusammengesetzten Zustand der Form auf korrespondierenden und genau passenden Auflageflächen des anderen Teils 12 der Form auf. Die Auflageflächen 23, und die Fläche des Rings 21 liegen alle in der Fugenebene der Form. Dabei können die Auflageflächen 23 theoretisch auch eine andere Höhe aufweisen als die Fläche des Rings 21. Die Fugenebene wäre dann entsprechend gewölbt geformt oder zumindest nicht eben und könnte dann auch geometrisch korrekter als Fugenfläche bezeichnet werden.

Benachbart zu den vorderen vier Auflageflächen 23 (in Figur 3 oben) liegt nach innen benachbart eine Negativform für den Zahnbogen 24 mit den Erhöhungen beziehungsweise Verdickungen. Die Negativformen für den Zahnbogen 24 sind der Bereich der inneren Oberfläche der Form, der zum Ausformen des Zahnbogens 2 nach Figur 1 vorgesehen ist. An der Vorderseite des Teils 10 der Form ist eine Negativform für die Markierung 26 vorgesehen. Die Negativformen für die Markierung 26 ist dementsprechend der Bereich der inneren Oberfläche der Form, der zum Ausformen der Markierung 6 nach Figur 1 vorgesehen ist.

In der Mitte und neben dem Rand 21 des Teils 10 der Form sind Öffnungen 27 zum Einfüllen von Material zur Herstellung des Prothesenbasisrohlings 1 nach Figur 1 oder eines anderen Prothesenbasisrohlings vorgesehen. Diese Öffnungen 27 münden in die Einfüllstutzen 14 nach Figur 2.

In die Negativform für den Zahnbogen 24 kann eine Einlage 28 eingesetzt und bevorzugt befestigt werden, mit der eine von der durch den Teil 10 der Form abweichende innere Oberfläche erzeugt werden kann. Die Einlage 28 weist dazu eine bereichsweise Verdickung 29 auf. Die Einlage 28 ist in Figur 3 durch gestrichelte Linien angedeutet, die anzeigen sollen, dass es sich um ein separates und herausnehmbares zusätzliches Stück der Form handelt. Bei Verwendung der Einlage 28 wird diese in der Negativform für den Zahnbogen 24 befestigt und dadurch die innere Oberfläche der Form in diesem Bereich verändert. Dadurch wird der mit der so modifizierten Form erzeugte Prothesenbasisrohling in diesem Bereich weniger dick sein, also weniger Material aufweisen und gegebenenfalls aufgrund der Verdickung 29 auch kürzer sein. Hierdurch lässt sich der Prothesenbasisrohling an die Mundsituation des Patienten anpassen.

In gleicher Weise kann auch ein anderes Teil aus einer Vielzahl von Teilen einer Form verwendet werden, um den Teil 10 und/oder den Teil 12 zu ersetzen. Auch dadurch lässt sich der mit der Form erzeugte Prothesenbasisrohling an die Mundsituation des Patienten anpassen.

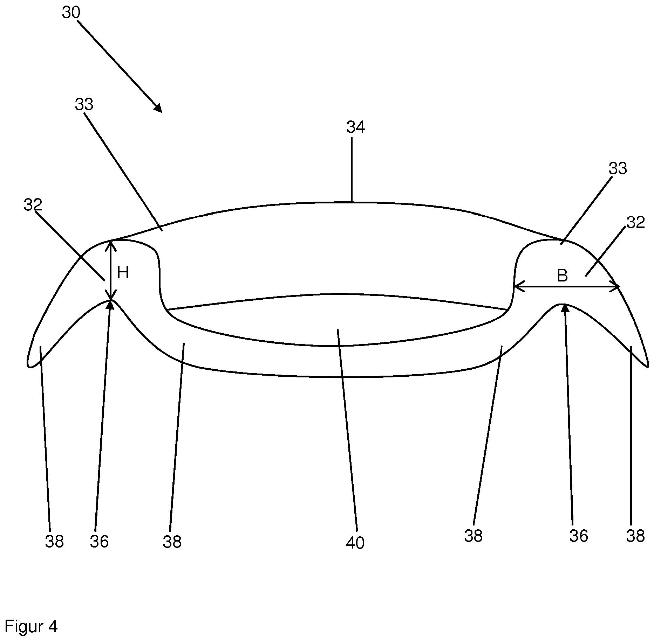

Figur 4 zeigt eine schematische seitliche Ansicht eines erfindungsgemäßen Prothesenbasisrohlings 30, der mit einer erfindungsgemäßen Form, wie beispielsweise einer Form nach den Figuren 2 und 3, beziehungsweise einem erfindungsgemäßen Verfahren, hergestellt wurde. Der Prothesenbasisrohling 30 weist, analog dem nach Figur 1, einen Zahnbogen 32 auf, dem eine Breite B zugeordnet werden kann. Der Zahnbogen 32 erstreckt sich randseitig in Parabelform entlang des Prothesenbasisrohlings 30. Der Zahnbogen 32 weist eine Verdickung 33 in okklusaler Richtung auf, die ausreichend Raum zur Ausbildung von Papillen in der Prothesenbasis vorsieht. Im Bereich eines Scheitelpunkts 34 des Prothesenbasisrohlings 1 ist die Höhe H des Zahnbogens 32 etwas stärker vergrößert.

An der Unterseite des Prothesenbasisrohlings 30 ist eine Vertiefung 36 vorgesehen (in Figur 4 unten), die als Vorformung einer Auflagefläche des zahnlosen Zahnfleischs des Patienten in der zu erzeugenden Prothesenbasis dient. Seitlich neben dem Zahnbogen 32 sind Verbreiterungen 38 beziehungsweise Überstände 38 vorgesehen, aus denen eine Verbindungsfläche zum Kiefersattel des Patienten geformt werden kann.

Ein Mittelstück 40 reicht bei dieser Ausführungsform des Prothesenbasisrohlings 30 bis zur Mitte, so dass aus diesem Prothesenbasisrohling 30 auch eine großflächige Gaumenabdeckung geformt werden kann.

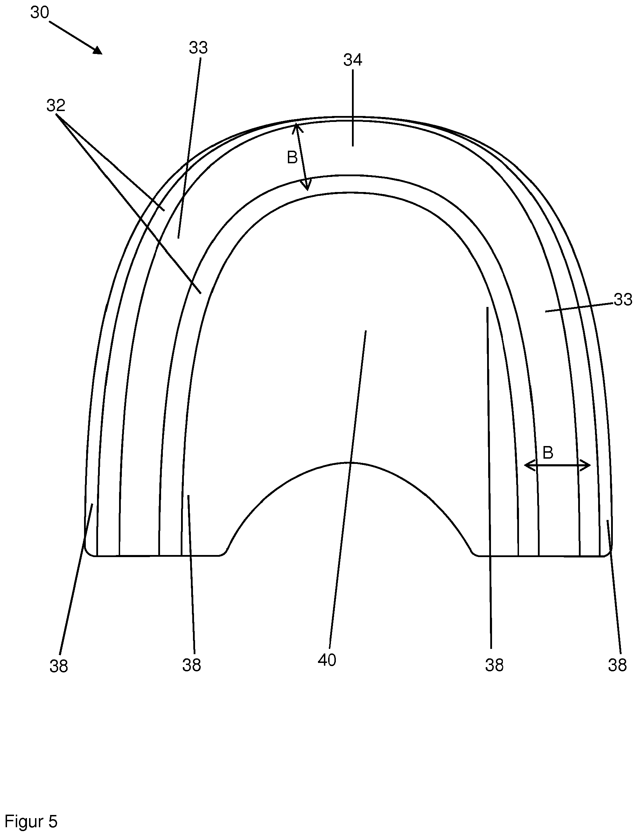

Figur 5 zeigt eine schematische Aufsicht auf einen weiteren erfindungsgemäßen Prothesenbasisrohling 30. Der Prothesenbasisrohling 30 weist abgerundete Ecken auf, um die Gefahr eines Abbrechens von Teilen des Prothesenbasisrohlings 30 bei der Bearbeitung zu verhindern. Solche Abrundungen können bei allen erfindungsgemäßen Prothesenbasisrohlingen 1, 30 vorgesehen sein.

Der Prothesenbasisrohling 30 weist eine randseitige parabelförmige Verdickung 33 eines Zahnbogens 32 auf, der sich analog der Ausführungen nach Figur 4 nach oben (in Figur 5 in Richtung des Betrachters) erstreckt und dort die Verdickung 33 in okklusaler Richtung bildet. Im Bereich des Scheitelpunkts 34 des Zahnbogens 32 kann die Höhe des Zahnbogens 32 beziehungsweise die Verdickung 33 größer sein als an den anderen Bereichen des Zahnbogens 32. Bei der Ausführung nach Figur 5 ist auch die Breite B im Bereich des Scheitelpunkts 34 etwas erhöht.

Neben dem Zahnbogen 32 sind Überstände 38 vorgesehen, die dem gleichen Zweck dienen wie die Überstände 38 nach Figur 4. In der Mitte des Prothesenbasisrohlings 30 ist ein Mittelstück 40 vorgesehen, aus dem eine Gaumenplatte oder eine sublinguale Auflage der zu erzeugenden Prothesenbasis herstellbar ist. Im Bereich des Scheitelpunkts 34 des Prothesenbasisrohlings 30 ist der labiale Überstand 38 stark reduziert oder nicht mehr vorhanden. Auch der Querschnitt des Zahnbogens 32 senkrecht zur Parabelkurve beziehungsweise senkrecht zum Zahnbogen 32 verändert sich entlang des Zahnbogens 32. Die Steigung der labialen Flanke des Zahnbogens 32 ist im Bereich der Scheitelpunkts 34 reduziert.

Die Prothesenbasisrohlinge 30 nach den Figuren 4 und 5 sind alle symmetrisch bezüglich der Sagittalebene aufgebaut, in der der Scheitelpunkt 34 liegt. Eventuell anatomisch notwendige Asymmetrien können beim Ausfräsen des Prothesenbasisrohlings 30 bei der Herstellung der Prothesenbasis erzeugt werden oder durch zusätzliche Teile der Form oder Einlagen der Form erzeugt werden.

Im Folgenden wird ein erfindungsgemäßes Verfahren anhand der Figuren 1 bis 5 erläutert.

Zur Herstellung einer Prothesenbasis wird ein Prothesenbasisrohling 1, 30 mit einer Form hergestellt, wobei die Teile 10, 12 und gegebenenfalls die Einlagen 28 der Form aus einer Vielzahl von Teilen und gegebenenfalls Einlagen unterschiedlicher Oberfläche ausgewählt werden. Dabei wird die passende innere Oberfläche 24 der Form rechnergestützt bestimmt. Die möglichen inneren Oberflächen 24 der verschiedenen Formen sind dazu elektronisch in einer Datenbank gespeichert. Die Mundsituation des zu behandelnden Patienten wird mit Hilfe eines 3D-Scanners eingescannt und digitalisiert. Anhand von diesen und gegebenenfalls von weiteren Daten wird ein virtuelles Modell der zu erzeugenden Prothesenbasis mit Hilfe eines CAD-Verfahrens erzeugt.

Anschließend wird getestet, in welchen oder in welche der vorhandenen möglichen inneren Oberflächen der Form die zu erzeugende Prothesenbasis passen könnte. Es wird eine Liste der möglichen Prothesenbasisrohlinge 1, 30 auf einem Bildschirm eines Computers angeboten. Die Reihenfolge kann sich nach der Volumendifferenz zwischen der zu erzeugenden Prothesenbasis und der inneren Oberfläche 24 der Form richten, wobei geringere Volumendifferenzen favorisiert werden. Alternativ kann auch nur die Form mit der geringsten Volumendifferenz angezeigt werden. Die geringe Volumendifferenz hat den Vorteil, dass bei mit diesen Formen hergestellten Prothesenbasisrohlingen 1, 30 weniger Material abgetragen werden muss als bei Prothesenbasisrohlingen 1, 30 mit einer höheren Volumendifferenz zu der zu erzeugenden Prothesenbasis.

Anschließend wird eine Form mit den passenden Teilen 10, 12 und gegebenenfalls mit passenden Einlagen 28 durch den Anwender oder vom Computer ausgewählt und alle Teile 10, 12 und gegebenenfalls Einlagen 28 werden zusammengesetzt. Die zusammengesetzte Form wird mit einem aushärtbaren Kunststoff gefüllt beziehungsweise gestopft und der Kunststoff in der Form ausgehärtet. Die Teile 10, 12 und gegebenenfalls die Einlage 28 werden von dem so erzeugten und ausgehärteten Prothesenbasisrohling 1, 30 gelöst. Wenn notwendig werden Grate entfernt. Der herausgelöste Prothesenbasisrohling 1, 30 wird in eine CAM-Vorrichtung, wie beispielsweise eine CAM-Vierachs-Fräse eingespannt. Dabei muss auf die richtige Orientierung des Prothesenbasisrohlings 1, 30 in der CAM-Vorrichtung geachtet werden. Hierzu können an dem Prothesenbasisrohling 1, 30 Markierungen 6 oder Ausformungen vorgesehen sein, die eine bestimmte Lage und Orientierung der Prothesenbasisrohlinge 1, 30 in der CAM-Vorrichtung ermöglichen oder bevorzugt erzwingen.

Anschließend wird auf der Basis des CAD-Modells der zu erzeugenden Prothesenbasis diese aus dem eingespannten Prothesenbasisrohling 1, 30 herausgefräst oder über ein anderes subtraktives CAM-Verfahren hergestellt.

In diese Prothesenbasis können auch gleich Ausnehmungen oder Flächen zur Aufnahme von Prothesenzähnen erzeugt werden. Anschließend werden Prothesenzähne in die Prothesenbasis eingeklebt und die Dental-Prothese ist fertiggestellt.

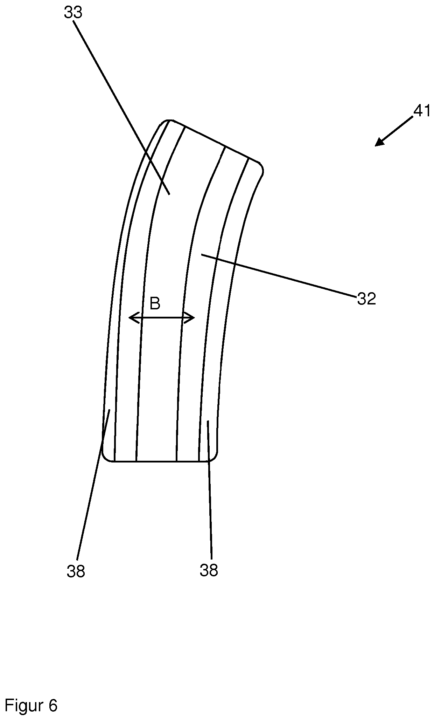

Figur 6 zeigt eine schematische Aufsicht auf einen erfindungsgemäßen Prothesenbasisrohling 41 für eine Teilprothese. Der Prothesenbasisrohling 41 stellt im Prinzip einen backenseitigen Teil des Prothesenbasisrohlings 30 nach Figur 5 dar. Ein Zahnbogenabschnitt 32 ist in Form eines Parabelabschnitts als Verdickung 33 des Materials des Prothesenbasisrohlings 41 vorgesehen. Da die Breite B des Prothesenbasisrohlings 41 fast so breit ist, wie der gesamte Querschnitt des Prothesenbasisrohlings 41, ist der Zahnbogen 32 auch bei dieser Ausführungsform randseitig angeordnet. Neben dem Zahnbogen 32 sind kurze Überstände 38 vorgesehen, die den gleichen Zweck erfüllen, wie die Überstände 38 der Ausführungsbeispiele nach den Figuren 4 und 5.

Auf der Unterseite des Prothesenbasisrohlings 41 kann eine Vertiefung vorgesehen sein (in der Aufsicht nach Figur 6 nicht zu sehen), die sich unterhalb der Verdickung 33 des Zahnbogens 32 in gleicher Richtung und Lage erstreckt. Die Vertiefung dient als Vorformung der späteren Auflagefläche auf das Zahnfleisch einer aus dem Prothesenbasisrohling 41 erzeugten Teil-Prothesenbasis beziehungsweise der daraus erzeugten Teil-Dentalprothese.

Das Herstellungsverfahren für den Prothesenbasisrohling 41 nach Figur 6 läuft analog dem zu den Figuren 1 bis 5 geschilderten Verfahren ab, wobei eben nur eine Teil-Dentalprothese erzeugt wird.

Die Prothesenbasisrohlinge 30, 41 nach den Figuren 4 bis 6 weisen erfindungsgemäß bevorzugt Stege (nicht gezeigt) und einen Ring (nicht gezeigt) auf, wie dies in Figur 1 dargestellt ist. Der Ring umgibt die in den Figuren 4 bis 6 gezeigten Stücke des Prothesenbasisrohlings 30, 41 und ist in der Transversalebene angeordnet. Der Ring kann als Halterung für eine CAM-Fräse verwendet werden. Zudem ist auf dem Ring bevorzugt eine Markierung (in den Figuren 4 bis 6 nicht gezeigt) vorgesehen, mit der die Orientierung und Lage des Prothesenbasisrohlings 30, 41 in der CAM-Fräse bestimmt werden kann. Der Ring und die Stege sind in den Figuren 4 bis 6 lediglich der Übersicht halber nicht dargestellt, für die Ausführung der vorliegenden Erfindung jedoch besonders bevorzugt.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 30: Prothesenbasisrohling
- 2, 32: Vorgeformter Zahnbogen
- 3: Ring
- 4: Steg
- 6: Markierung
- 8: Gaumenplatte / Sublinguale Platte
- 10: Formteil
- 12: Formteil
- 14: Einfüllstutzen
- 16: Aufnahme für die okklusale Erhöhung auf dem Zahnbogen
- 18: Aufnahme für Auflage auf Zahnfleisch
- 20: Rand
- 21: Negativform für Ring
- 22: Negativform für Steg
- 23: Auflagefläche
- 24: Negativform für Zahnbogen
- 26: Negativform für Markierung
- 27: Einfüllöffnung
- 28: Einlage
- 29: Verdickung
- 33: Okklusale Verdickung
- 34: Scheitelpunkt des Zahnbogens
- 36: Vertiefung an der Auflagefläche zum Zahnfleisch
- 38: Überstand
- 40: Mittelstück
- 41: Prothesenbasisrohling für Teilprothese
- B: Breite des Zahnbogens
- H: Höhe des Zahnbogens

## Patentansprüche

1. Form zur Herstellung eines vorgeformten Prothesenbasisrohlings (1, 30, 41), wobei der vorgeformte Prothesenbasisrohling (1, 30, 41) zur Herstellung einer Prothesenbasis für eine Dentalprothese mit einem subtraktiven CAM-Verfahren vorgesehen ist, wobei die Form eine innere Oberfläche (21, 22, 24, 26) aufweist, die ein Negativ des zu erzeugenden Prothesenbasisrohlings (1, 30, 41) ist, wobei die innere Oberfläche (21, 22, 24, 26) ein Negativ eines Zahnbogens (2, 32) oder eines Zahnbogenabschnitts (32) darstellt, wobei der Zahnbogen (2, 32) oder der Zahnbogenabschnitt (32) eine Verdickung (33) in okklusaler Richtung aufweist, und die Form aus zumindest zwei Teilen (10, 12) besteht, die voneinander lösbar aneinander befestigt sind oder die lösbar aneinander befestigbar sind, **dadurch gekennzeichnet, dass**
die innere Oberfläche (21, 22, 24, 26) der Form neben dem Zahnbogen (2, 32) oder dem Zahnbogenabschnitt (32) zusätzlich Stege (4) und eine Halterung (3) für den Prothesenbasisrohling (1, 30, 41) definiert, wobei die Stege (4) die Halterung (3) mit dem Zahnbogen (2, 32) oder dem Zahnbogenabschnitt (32) verbinden.

2. Form nach Anspruch 1, **dadurch gekennzeichnet, dass**
die Form ein Set einer Vielzahl von Teilen (10, 12) zur Herstellung unterschiedlicher Prothesenbasisrohlinge ist und sich die innere Oberfläche (21, 22, 24, 26) der Form durch einen Austausch von Teilen (10, 12) ausgewählt aus der Vielzahl unterschiedlicher Teile (10, 12) für gleiche Teilbereiche der Form anpassen lässt, wobei die gegeneinander austauschbaren Teile (10, 12) einen abweichenden Teilbereich der inneren Oberfläche (21, 22, 24, 26) der Form zur Herstellung unterschiedlicher Prothesenbasisrohlinge (1, 30, 41) aufweisen.

3. Form nach Anspruch 1 oder 2, **dadurch gekennzeichnet, dass** die Form mehrere austauschbare Teile (10, 12) und/oder innerhalb der Form einsetzbare Einlagen (28) aufweist, die die innere Oberfläche (21, 22, 24, 26) bei einer Verwendung der Teile (10, 12) und/oder Einlagen (28) zum Zusammensetzen der Form verändern.

4. Form nach einem der vorangehenden Ansprüche, **dadurch gekennzeichnet, dass** die Halterung (3), die durch die innere Oberfläche (21) der Form definiert ist, einen Ring (3) um den zu erzeugenden Zahnbogen (2, 32) oder Zahnbogenabschnitt (32) bildet.

5. Form nach Anspruch 4, **dadurch gekennzeichnet, dass**
der Zahnbogen (2, 32) oder der Zahnbogenabschnitt (32) über wenigstens drei Stege (4) mit dem Ring (3) verbunden ist.

6. Form nach einem der vorangehenden Ansprüche, **dadurch gekennzeichnet, dass** die zumindest zwei Teile (10, 12) der Form in zumindest einer Fugenebene aneinander in Passform anliegen, wobei alle der durch die Form zu erzeugenden Stege (4) in der zumindest einen Fugenebene der Form angeordnet sind.

7. Form nach einem der vorangehenden Ansprüche, **dadurch gekennzeichnet, dass** die innere Oberfläche (21, 22, 24, 26) der Form eine Ausnehmung (6) oder einen Vorsprung (6) definiert, der als Referenzmarkierung (6) zur Bestimmung der Orientierung des Prothesenbasisrohlings (1, 30, 41) verwendbar ist, wobei bevorzugt die innere Oberfläche (21, 22, 24, 26) der Form eine Ausnehmung (6) oder einen Vorsprung (6) auf der Halterung (3) definiert, insbesondere auf einem Ring (3) als Halterung (3) definiert.

8. Form nach einem der vorangehenden Ansprüche, **dadurch gekennzeichnet, dass** die Form aus Metall, Silikon oder Dubliergel besteht, bevorzugt aus Metall besteht, besonders bevorzugt aus Stahl, Aluminium oder Messing besteht.

9. Form nach einem der vorangehenden Ansprüche, **dadurch gekennzeichnet, dass** die zumindest zwei Teile (10, 12) entlang einer Transversalebene aneinandergefügt sind oder aneinander zu fügen sind.

10. Form nach einem der vorangehenden Ansprüche, **dadurch gekennzeichnet, dass** die innere Oberfläche (21, 22, 24, 26) der Teile (10, 12) der Form keinen geometrischen Körper bildet, der beim Entformen des Prothesenbasisrohlings (1, 30, 41) das Lösen der Form von dem ausgehärteten Prothesenbasisrohling (1, 30, 41) verhindert, wobei bevorzugt keine Unterschneidungen oder unter sich gehende Bereiche in der inneren Oberfläche (21, 22, 24, 26) der Teile (10, 12) der Form vorgesehen sind.

11. Verfahren zur Herstellung eines Prothesenbasisrohlings mit einer Form nach einem der vorangehenden Ansprüche, bei dem
ein Heißpolymerisat oder ein Kaltpolymerisat in die zumindest zweiteilige, zusammengesetzte Form gefüllt wird, anschließend das Heißpolymerisat oder das Kaltpolymerisat aushärtet und daran anschließend die Teile (10, 12) der Form von dem ausgehärteten Prothesenbasisrohling (1, 30, 41) gelöst werden.

12. Verfahren nach Anspruch 11, **dadurch gekennzeichnet, dass**
der Prothesenbasisrohling (1, 30, 41) zumindest bereichsweise entgratet wird, nachdem die Teile (10, 12) der Form abgelöst wurden.

13. Verfahren nach Anspruch 11 oder 12, **dadurch gekennzeichnet, dass** ein Heißpolymerisat in die zumindest zweiteilige, zusammengesetzte Form aus Metall mit einer Stopf-Press-Technik oder einer Injektionstechnik gefüllt wird.

14. Verfahren nach Anspruch 11 oder 12, **dadurch gekennzeichnet, dass** ein Kaltpolymerisat in die zumindest zweiteilige, zusammengesetzte Form aus Metall, Silikon oder Dubliergel mit einer Gieß-Technik oder einer Injektionstechnik gefüllt wird.

15. Prothesenbasisrohling hergestellt mit einem Verfahren nach einem der Ansprüche 11 bis 14 oder hergestellt mit Hilfe einer Form nach einem der Ansprüche 1 bis 10, wobei der Prothesenbasisrohling (1, 30, 41) einen Zahnbogen (2, 32) oder einen Zahnbogenabschnitt (32) mit einer Verdickung (33) in okklusaler Richtung und Stege (4) und eine Halterung (3) für den Prothesenbasisrohling (1, 30, 41) aufweist, wobei die Stege (4) die Halterung (3) mit dem Zahnbogen (2, 32) oder dem Zahnbogenabschnitt (32) verbinden.

16. Prothesenbasisrohling nach Anspruch 15, **dadurch gekennzeichnet, dass** die Stege (4) mit einem Ring (3) als Halterung (3) verbunden sind und die den Zahnbogen (2, 32) oder den Zahnbogenabschnitt (32) im Inneren des Rings (3) halten.

17. Prothesenbasisrohling nach Anspruch 15 oder 16, **dadurch gekennzeichnet, dass** der Prothesenbasisrohling (1, 30, 41) Grate aufweist, die von den Verbindungen der zumindest zwei Teile (10, 12) der Form verursacht werden.

## Claims

1. A form for producing a pre-moulded prosthesis base blank (1, 30, 41), wherein the pre-moulded prosthesis base blank (1, 30, 41) is provided for producing a prosthesis base for a dental prosthesis with a subtractive CAM method, wherein the form features an inner surface (21, 22, 24, 26) which is a negative of the prosthesis base blank (1, 30, 41) to be produced, wherein the inner surface (21, 22, 24, 26) represents a negative of a dental arch (2, 32) or a dental arch section (32), wherein the dental arch (2, 32) or the dental arch section (32) comprises a thickening (33) in the occlusal direction, and the form consists of at least two parts (10, 12), which are affixed in such a manner that they are detachable from each other or which are detachably affixable to each other, **characterized in that**
the inner surface (21, 22, 24, 26) of the form next to the dental arch (24) or dental arch section additionally defines bridges (4) and a retaining fixture (3) for the prosthesis base blank (1, 30, 41), wherein the bridges (4) connect the retaining fixture (3) with the dental arch (2, 32).

2. The form according to Claim 1, **characterized in that**
the form is a set of a multitude of parts (10, 12) for producing different prosthesis base blanks and the inner surface (21, 22, 24, 26) of the form can be adapted by replacing parts (10, 12) selected from the multitude of different parts (10, 12) for the same parts of the form, wherein the parts that can replace each other (10, 12) have a different partial area of the inner surface (21, 22, 24, 26) of the form for producing different prosthesis base blanks (1, 30, 41).

3. The form according to Claim 1 or 2, **characterized in that**
the form features several replaceable parts (10, 12) and/or inserts (28) that can be inserted within the form, which change the inner surface (21, 22, 24, 26) when the parts (10, 12) and/or inserts (28) are used to compile the form.

4. The form according to any one of the preceding claims, **characterized in that** the retaining fixture (3) which is defined by the inner surface (21) of the form creates a ring (3) around the dental arch (2, 32) or dental arch section (32) to be produced.

5. The form according to claim 4, **characterized in that**
the dental arch (2, 32) or dental arch section (32) is connected to the ring (3) via at least three bridges (4).

6. The form according to any one of the preceding claims, **characterized in that** the at least two parts (10, 12) of the form lie in contact on each other in a perfect fit in at least one join plane, wherein all the bridges (4) to be produced by the form are arranged in the at least one join plane of the form.

7. The form according to any one of the preceding claims, **characterized in that** the inner surface (21, 22, 24, 26) of the form defines a recess (6) or protrusion (6) which can be used as a reference mark (6) to determine the orientation of the prosthesis base blank (1, 30, 41), wherein preferably, the inner surface (21, 22, 24, 26) of the form defines a recess (6) or a protrusion (6) on the retaining fixture (3), in particular on a ring (3) as the fixture (3).

8. The form according to any one of the preceding claims, **characterized in that** the form consists of metal, silicon or doubling gel, preferably of metal, particularly preferably of steel, aluminum or brass.

9. The form according to any one of the preceding claims, **characterized in that** the at least two parts (10, 12) are joined to each other or are to be joined to each other along the transversal plane.

10. The form according to any one of the preceding claims, **characterized in that** the inner surface (21, 22, 24, 26) of the parts (10, 12) of the form does not create a geometric body, which, when the prosthesis base blank (1, 30, 41) is demoulded, prevents the detachment of the form from the hardened prosthesis base blank (1, 30, 41), wherein preferably, no undercutting or undercut areas in the inner surface (21, 22, 24, 26) of the parts (10, 12) of the form are provided.

11. A method for producing a prosthesis base blank with a form according to any one of the preceding claims, in which
a hot polymer or a cold polymer is filled into the at least two-part compiled form, then the hot polymer or the cold polymer is hardened, and as a next step, the parts (10, 12) of the form are detached from the hardened prosthesis base blank (1, 30, 41).

12. The method according to Claim 11, **characterized in that**
The prosthesis base blank (1, 30, 41) is deburred at least in sections after the parts (10, 12) of the form have been detached.

13. The method according to Claim 11 or 12, **characterized in that** a hot polymer is filled into the at least two-part compiled metal form using press and pack technique or injection technique.

14. The method according to Claim 11 or 12, **characterized in that** a cold polymer is filled into the at least two-part compiled metal, silicon or doubling gel form using pouring technique or injection technique.

15. A prosthesis base blank produced using a method according to any one of Claims 11 to 14, or produced with the aid of a form according to any one of Claims 1 to 10, wherein the prosthesis base blank (1, 30, 41) comprises a dental arch (2, 32) or a dental arch section (32) comprising a thickening (33) in the occlusal direction and bridges (4) and a retaining fixture (3) for the prosthesis base blank (1, 30, 41), wherein the bridges (4) connect the retaining fixture (3) with the dental arch (2, 32) or the dental arch section (32).

16. The prosthesis base blank according to Claim 15, **characterized in that** the bridges (4) are connected to a ring (3) as a retaining fixture (3), and which hold the dental arch (2, 32) or dental arch section (32) in the interior of the ring (3).

17. The prosthesis base blank according to Claim 15 or 16, **characterized in that** the prosthesis base blank (1, 30, 41) comprises burrs caused by the connections of the at least two parts (10, 12) of the form.

## Revendications

1. Moule pour la fabrication d'une ébauche de base de prothèse (1, 30, 41) préformée, où l'ébauche de base de prothèse (1, 30, 41) préformée est prévue pour la fabrication d'une base de prothèse pour une prothèse dentaire avec un procédé FAQ soustractif, où le moule présente une surface intérieure (21, 22, 24, 26) qui est un négatif de l'ébauche de base de prothèse (1, 30, 41) à créer, où la surface intérieure (21, 22, 24, 26) représente un négatif d'une arcade dentaire (2, 32) ou d'un segment d'arcade dentaire (32), où l'arcade dentaire (2, 32) ou le segment d'arcade dentaire (32) présente un épaississement (33) dans la direction occlusale, et le moule est constitué d'au moins deux parties (10, 12) qui sont fixées l'une à l'autre de manière amovible ou qui peuvent être fixées l'une à l'autre de manière amovible, **caractérisé en ce que** la surface intérieure (21, 22, 24, 26) du moule définit en outre des tiges (4) et un support (3) pour l'ébauche de base de prothèse (1, 30, 41) à côté de l'arcade dentaire (2, 32) ou du segment d'arcade dentaire (32), où les tiges (4) relient le support (3) avec l'arcade dentaire (2, 32) ou le segment d'arcade dentaire (32).

2. Moule selon la revendication 1, **caractérisé en ce que**
le moule est un ensemble d'une multiplicité de parties (10, 12) pour la fabrication d'ébauches de bases de prothèses différentes et la surface intérieure (21, 22, 24, 26) du moule est à adapter pour des zones partielles identiques du moule par un échange de parties (10, 12) choisies dans la multiplicité des parties (10, 12) différentes, où les parties (10, 12) échangeables entre elles présentent une zone partielle dérivée de la surface intérieure (21, 22, 24, 26) du moule pour la fabrication d'ébauches de bases de prothèses (1, 30, 41) différentes.

3. Moule selon la revendication 1 ou la revendication 2, **caractérisé en ce que** le moule présente plusieurs parties (10, 12) échangeables et/ou des parties d'insertion pouvant être insérées à l'intérieur du moule qui modifient la surface intérieure (21, 22, 24, 26) lors de l'utilisation des parties (10, 12) et/ou des parties d'insertion (28) pour l'assemblage du moule.

4. Moule selon l'une des revendications précédentes, **caractérisé en ce que** le support (3), qui est défini par la surface intérieure (21) du moule, forme un anneau (3) autour de l'arcade dentaire (2, 32) ou du segment d'arcade dentaire (32) à créer.

5. Moule selon la revendication 4, **caractérisé en ce que**
l'arcade dentaire (2, 32) ou le segment d'arcade dentaire (32) est relié avec l'anneau (3) par le biais d'au moins trois tiges (4).

6. Moule selon l'une des revendications précédentes, **caractérisé en ce que** les au moins deux parties (10, 12) du moule sont adjacentes au niveau de l'ajustement de l'une par rapport à l'autre dans au moins un plan de jonction, où toutes les tiges (4) à créer par le moule sont disposées dans au moins un plan de jonction du moule.

7. Moule selon l'une des revendications précédentes, **caractérisé en ce que** la surface intérieure (21, 22, 24, 26) du moule définit une cavité (6) ou une protubérance (6) qui peut être employée en tant que repère de référence (6) pour la détermination de l'orientation de l'ébauche de base de prothèse (1, 30, 41), où de préférence la surface intérieure (21, 22, 24, 26) du moule définit une cavité (6) ou une protubérance (6) sur le support (3), notamment, définit sur un anneau (3) servant de support (3).

8. Moule selon l'une des revendications précédentes, **caractérisé en ce que** le moule est constitué de métal, de silicone ou de gel de doublage, est constitué de préférence de métal, est constitué de manière particulièrement préférée en acier, en aluminium ou en laiton.

9. Moule selon l'une des revendications précédentes, **caractérisé en ce que** les au moins deux parties (10, 12) sont mises bout à bout ou sont à mettre bout à bout le long d'un plan transversal.

10. Moule selon l'une des revendications précédentes, **caractérisé en ce que** la surface intérieure (21, 22, 24, 26) des parties (10, 12) du moule ne forme pas de corps géométrique qui empêche le détachement du moule de l'ébauche de base de prothèse (1, 30, 41) lors du démoulage de l'ébauche de base de prothèse (1, 30, 41), où de préférence aucune sous-découpe ou zone allant en-dessous ne sont prévues dans la surface intérieure (21, 22, 24, 26) des parties (10, 12) du moule.

11. Procédé de fabrication d'une ébauche de base de prothèse avec un moule selon l'une des revendications précédentes, chez lequel
un produit de polymérisation à chaud ou un produit de polymérisation à froid est versé dans le moule assemblé en au moins deux parties, ensuite, le produit de polymérisation à chaud ou le produit de polymérisation à froid durcit et ensuite, encore, les parties (10, 12) du moule sont détachées de l'ébauche de base de prothèse (1, 30, 41).

12. Procédé selon la revendication 11, **caractérisé en ce que**
l'ébauche de base de prothèse (1, 30, 41) est ébavurée au moins par endroits après que les parties (10, 12) du moule aient été détachées.

13. Procédé selon la revendication 11 ou la revendication 12, **caractérisé en ce qu'**un produit de polymérisation à chaud est versé dans le moule assemblé en au moins deux parties avec une technique de bourrage et de pressage ou une technique d'injection.

14. Procédé selon la revendication 11 ou la revendication 12, **caractérisé en ce qu'**un produit de polymérisation à froid est versé dans le moule assemblé en au moins deux parties, en métal, en silicone ou en gel de doublage, avec une technique de coulage ou une technique d'injection.

15. Ebauche de base de prothèse fabriquée avec un procédé selon l'une des revendications 11 à 14 ou fabriquée à l'aide d'un moule selon l'une des revendications 1 à 10, où l'ébauche de base de prothèse (1, 30, 41) présente une arcade dentaire (2, 32) ou un segment d'arcade dentaire (32) avec un épaississement (33) en direction occlusale et des tiges (4) et un support (3) pour l'ébauche de base de prothèse (1, 30, 41), où les tiges (4) relient le support (3) avec l'arcade dentaire (2, 32) ou avec le segment d'arcade dentaire (32).

16. Ebauche de base de prothèse selon la revendication 15, **caractérisée en ce que** les tiges (4) sont reliées avec un anneau (3) servant de support (3) et qui tiennent l'arcade dentaire (2, 32) ou le segment d'arc dentaire (32) à l'intérieur de l'anneau (3).

17. Ebauche de base de prothèse selon la revendication 15 ou la revendication 16, **caractérisée en ce que**
l'ébauche de base de prothèse (1, 30, 41) présente des arêtes qui sont occasionnées par les liaisons des au moins deux parties (10, 12) du moule.
